(19) Europäisches Patentamt
European Patent Office
Office européen des brevets

(11) Veröffentlichungsnummer: **0 132 580**
**A1**

(12) # EUROPÄISCHE PATENTANMELDUNG

(21) Anmeldenummer: 84106954.5

(22) Anmeldetag: 18.06.84

(51) Int. Cl.⁴: C 07 D 207/16
C 07 D 209/42, C 07 D 209/52
C 07 D 209/54

(30) Priorität: 23.06.83 DE 3322530

(43) Veröffentlichungstag der Anmeldung: 13.02.85 Patentblatt 85/7

(84) Benannte Vertragsstaaten: AT BE CH DE FR GB IT LI LU NL SE

(71) Anmelder: HOECHST AKTIENGESELLSCHAFT
Postfach 80 03 20
D-6230 Frankfurt am Main 80(DE)

(72) Erfinder: Henning, Rainer, Dr.
Rotenhofstrasse 31
D-6234 Hattersheim am Main(DE)

(72) Erfinder: Urbach, Hansjörg, Dr.
Le Levandoustrasse 41
D-6242 Kronberg/Taunus(DE)

(54) **Verfahren zur Herstellung von mono-, bi- und tricyclischen Aminosäuren.**

(57) Die Erfindung betrifft ein Verfahren zur Herstellung von Verbindungen der Formel I

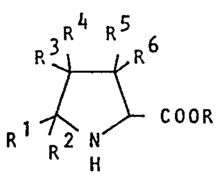

in welcher R für Wasserstoff, Alkyl oder Aralkyl steht, und $R^1$ bis $R^6$ gleiche oder verschiedene Reste Wasserstoff, Alkyl, Cycloalkyl, Cycloalkylalkyl, Cycloalkenyl-alkyl, Aralkyl oder Aryl, beide jeweils im Arylteil durch Alkyl, Alkoxy, Hydroxy, Halogen, Nitro, Methylendioxy und/oder Cyano mono-, di- oder trisubstituiert, bedeuten oder in welcher

zwei der Reste $R^1$ bis $R^6$ zusammen mit dem (den) sie tragenden Kohlenstoffatom(en) mono- oder bicyclisches Ringsystem bilden und die übrigen Reste Wasserstoff sind, dadurch gekennzeichnet, daß man ein Pyrrolidinderivat der Formel II mit einem Oxidationsmittel in Gegenwart eines Silbersalzes in ein $\Delta^1$-Pyrrolinderivat der Formel III überführt, dieses mit Cyanwasserstoff oder einem Metallcyanid zu einem Nitril der Formel IV umsetzt, und dieses einer Solvolyse mit einer Verbindung der Formel ROH unterwirft.

EP 0 132 580 A1

Croydon Printing Company Ltd.

Verfahren zur Herstellung von mono-, bi- und tricyclischen Aminosäuren

Die Erfindung betrifft ein Verfahren zur Herstellung von Verbindungen der Formel I

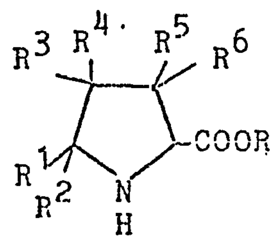

I

in welcher

R für Wasserstoff, $(C_1-C_6)$-Alkyl oder $(C_7-C_9)$-Aralkyl steht, und

$R^1$ bis $R^6$ gleich oder verschieden sind und unabhängig voneinander Wasserstoff, $(C_1-C_8)$-Alkyl, $(C_3-C_9)$-Cycloalkyl, $(C_3-C_9)$-Cycloalkyl-$(C_1-C_4)$-alkyl, $(C_5-C_9)$-Cycloalkenyl-$(C_1-C_4)$-alkyl, $(C_6-C_{12})$-Aryl-$(C_1-C_4)$-alkyl oder $(C_6-C_{12})$-Aryl, die beide jeweils im Arylteil durch $(C_1-C_4)$-Alkyl, $(C_1-C_4)$-Alkoxy, Hydroxy, Halogen, Nitro, Methylendioxy und/oder Cyano mono-, di- oder trisubstituiert sein können, bedeuten

oder in welcher

zwei der Reste $R^1$ bis $R^6$ zusammen mit dem sie tragenden Kohlenstoffatom bzw. mit den beiden sie tragenden Kohlenstoffatomen einen 4- bis 10-gliedriges gesättigtes oder ungesättigtes mono- oder bicyclisches carbocyclisches Ringsystem bilden und die übrigen Reste Wasserstoff sind,

das dadurch gekennzeichnet ist, daß man ein Pyrrolidinderivat der Formel II,

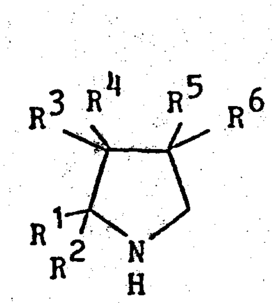

II

in welcher $R^1$ bis $R^6$ die gleiche Bedeutung wie in Formel I haben, mit einem Oxidationsmittel in Gegenwart eines Silbersalzes in ein $\Delta^1$-Pyrrolinderivat der Formel III,

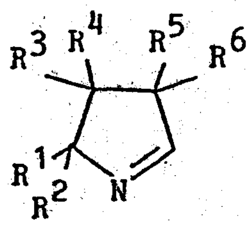

III

in welcher $R^1$ bis $R^6$ die gleiche Bedeutung wie in Formel I haben, überführt, dieses mit Cyanwasserstoff oder einem Cyanid zu einer Verbindung der Formel IV,

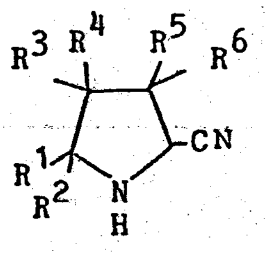

IV

in welcher $R^1$ bis $R^6$ die gleiche Bedeutung wie in Formel I haben, umsetzt, und diese mit einer Verbindung der Formel ROH, in welcher R die oben definierte Bedeutung hat, unter Bildung einer Verbindung der Formel I umsetzt.

Aus der Literatur ist die Herstellung von Prolinderivaten durch Blausäureaddition an $\Delta^1$-Pyrrolinderivate und anschließende Hydrolyse in einigen wenigen Fällen bekannt, die benötigten $\Delta^1$-Pyrrolinderivate werden jedoch größtenteils auf sehr umständlichen Wegen hergestellt (z. B. J. Chem.

Soc. 1959, 2087). Die Oxidation von cyclischen Aminen zu den entsprechenden Iminen ist nach Angaben in der Literatur zumeist eine ungünstige Reaktion. Das Verfahren leidet darunter, daß man toxische Reagenzien (Quecksilbersalze) verwenden muß und die Ausbeuten größtenteils sehr niedrig sind (J. Chem. Soc. 1959, 2087). Ausnahmen sind hierbei Verbindungen mit tertiären Stickstoffatomen (z. B. J. Amer. Chem. Soc. 79, 5279 (1957). Deren Oxidation liefert aber Iminderivate, die aufgrund sterischer Hinderung keine Blausäure addieren (J. Chem. Soc. 1959, 2087). Darüber hinaus gelingt bei sekundären Aminen die Oxidation nur mit wenigen ausgewählten Substituenten am fünfgliedrigen Ring. Aus J. Chem. Soc., Perkin Trans. I 1982, 3031 ist die Peroxodisulfat-Oxidation von Pyrrolidin zum $\Delta^1$-Pyrrolin-Trimeren bekannt.

Man kann ausgehend von Verbindungen der Formel IV auf mehrstufigen Synthesewegen zu Verbindungen der Formel I gelangen, für die jeweils wenige Beispiele in der Literatur beschrieben sind. Als solche Synthesewege kommen die elektrochemische Oxidation und anschließende Umsetzung mit Phenylisonitril (Tetrahedron Lett. 1981, 2411) oder Trimethylsilylcyanid (Tetrahedron Lett. 1981, 141), jeweils unter Lewis-Säurekatalyse und anschließende Hydrolyse oder Chlorierung in α-Stellung zum Stickstoff, Cyanidaustausch und Hydrolyse (EP-A-22 208) in Betracht.

Das erfindungsgemäße Verfahren zeichnet sich besonders durch einfache Durchführbarkeit der einzelnen Schritte und Verwendung billiger und leicht zugänglicher Reagenzien aus. Eine bevorzugte Ausführungsform ist dadurch gekennzeichnet, daß Verbindungen der Formel I hergestellt werden, in welcher

R die obengenannte Bedeutung hat

$R^1$ bis $R^6$ gleich oder verschieden sind und unabhängig voneinander Wasserstoff, Methyl, Ethyl, Propyl, Isopropyl, tert. Butyl, Cyclopentyl, Cyclohexyl,

Cycloheptyl, Cyclopentylmethyl, Cyclopentylethyl, Cyclohexylmethyl, Phenyl, Naphthyl, 4-Methoxyphenyl, 4-Fluorphenyl, 4-Chlorphenyl, 3,4-Dimethoxyphenyl, 3,4-Methylendioxyphenyl, 3,4-Dichlorphenyl, p-Tolyl, Benzyl, 4-Methoxybenzyl, 4-Chlorbenzyl, Phenylethyl, 2-Phenylpropyl oder 1-Phenylpropyl bedeuten

oder in welcher zwei der Reste $R^1$ bis $R^6$ in der oben definierten Weise einen Cyclobutyl-, Cyclopentyl-, Cyclohexyl-, Cycloheptyl-, Cyclooctyl-,Bicyclo/2.2.1/heptyl- oder Bicyclo/2.2.2/octyl-Ring bilden, wobei die Bindungen vorzugsweise vom gleichen Kohlenstoffatom oder von benachbarten Kohlenstoffatomen ausgehen und wobei die übrigen Reste Wasserstoff sind.

Besonders bevorzugt ist eine Ausführungsform, die dadurch gekennzeichnet ist, daß Verbindungen der Formel I hergestellt werden, in welcher

R die obengenannte Bedeutung hat, insbesondere aber Wasserstoff, tert. Butyl oder Benzyl bedeutet und
$R^1$ bis $R^6$ Wasserstoff bedeuten
oder worin
einer oder zwei der Rest $R^1$ bis $R^6$ unabhängig voneinander Methyl, Ethyl, Propyl, Isopropyl, Cyclopentyl, Cyclohexyl, Phenyl, 4-Methoxyphenyl, 4-Fluorphenyl, Benzyl, Phenethyl oder 4-Methoxybenzyl und die übrigen Wasserstoff bedeuten
oder
zwei der Reste $R^1$ bis $R^6$, die am gleichen oder an benachbarten Kohlenstoffatomen stehen, mit diesen zusammen einen Cyclopentyl-, Cyclohexyl-, Cycloheptyl-, Bicyclo/2.2.1/heptyl oder Bicyclo/2.2.2/octyl-Ring bilden, wobei die übrigen Reste Wasserstoff bedeuten.

Das erfindungsgemäße Verfahren liefert je nach Durchführung und Art der Substituenten $R^1$ bis $R^6$ die Verbindungen der Formel I als Gemische von Enantiomeren oder Diastereomeren oder als reine Diastereomere. Enstehende Gemische können durch geeignete, an sich bekannte Verfahren wie fraktionierte Kristallisation oder Chromatographie für Diastereomere, oder Bildung von diastereomeren Salzen, gegebenenfalls von geeigneten Derivaten, für Enantiomerengemische in die Bestandteile getrennt werden. Diese Trennung kann gegebenenfalls auch auf der Stufe der Verbindungen der Formel IV durchgeführt werden. Ganz besonders vorteilhaft können nach dem erfindungsgemäßen Verfahren die folgenden Verbindungen der Formel I hergestellt werden.

Prolin
cis-Octahydroindol-2-exo-carbonsäure
cis-Octahydroindol-2-endo-carbonsäure
trans-Octahydroindol-2-α-carbonsäure
trans-Octahydroindol-2-ß-carbonsäure
cis-Octahydrocyclopenta[b]pyrrol-2-exo-carbonsäure
cis-Octahydrocyclopenta[b]pyrrol-2-endo-carbonsäure
trans-Octahydrocyclopenta[b]pyrrol-2-α-carbonsäure
trans-Octahydrocyclopenta[b]pyrrol-2-ß-carbonsäure
2-Aza-spiro[4,5]decan-3-carbonsäure
2-Aza-spiro[4,4]nonan-3-carbonsäure
Spiro[bicyclo[2.2.2]octan-2,3'-pyrrolidin]-5-exo-carbonsäure
Spiro[bicyclo[2.2.2]octan-2,3'-pyrrolidin]-5-endo-carbonsäure
Spiro[bicyclo[2.2.1]heptan-2,3'-pyrrolidin]-5-exo-carbonsäure
cis-exo-3-Azatricyclo[$5.2.1.0^{2,6}$]decan-4-exo-carbonsäure
cis-exo-3-Azatricyclo[$5.2.1.0^{2,6}$]decan-4-endo-carbonsäure
cis-endo-3-Azatricyclo[$5.2.1.0^{2,6}$]decan-4-endo-carbonsäure
cis-endo-3-Azatricyclo[$5.2.1.0^{2,6}$]decan-4-exo-carbonsäure
cis-Decahydrocyclohepta[b]pyrrol-2-exo-carbonsäure
cis-Decahydrocyclohepta[b]pyrrol-2-endo-carbonsäure
trans-Decahydrocyclohepta[b]pyrrol-2-α-carbonsäure
trans-Decahydrocyclohepta[b]pyrrol-2-ß-carbonsäure

cis-Octahydroisoindol-1-exo-carbonsäure
cis-Octahydroisoindol-1-endo-carbonsäure
trans-Octahydroisoindol-1-α-carbonsäure
trans-Octahydroisoindol-1-ß-carbonsäure
cis-Octahydrocyclopenta [c] pyrrol-1-exo-carbonsäure
1-Aza-spiro[4.5]decan-2-carbonsäure
1-Aza-spiro[4.4]nonan-2-carbonsäure
4.5-cis-Diethylprolin
4.5-cis-Dimethylprolin
5.5-Dimethylprolin
4.4-Dimethylprolin
4.4-Diethylprolin
3.3-Dimethylprolin
4.5-cis-Diphenylprolin
4-Phenylprolin

sowie die Ester der obengenannten Aminosäuren.

Die Umsetzung des Pyrrolidins der Formel II zum $\Delta^1$-Pyrrolin der Formel III wird mit geeigneten Oxidationsmitteln durchgeführt, vorzugsweise mit Ammonium-, Alkali-, oder Erdalkaliperoxodisulfaten, besonders Natrium- oder Kaliumperoxodisulfat unter Katalyse von Silbersalzen, vorzugsweise Silbernitrat, die in Mengen von 0.1 bis 5 Mol-% zugesetzt werden. Die Reaktion wird in protisch polaren Lösungsmitteln, vorzugsweise in wäßriger Lösung bei -20° bis +80°C, vorzugsweise bei 0 bis +30°C durchgeführt.

Die Addition von Blausäure an Verbindungen der Formel III wird so durchgeführt, daß man eine Suspension oder Lösung der Verbindung der Formel III in einem protisch polaren Lösungsmittel, vorzugsweise Wasser, mit einem Alkali-, Erdalkali- oder Übergangsmetallcyanid, bevorzugt Natrium- oder Kaliumcyanid versetzt und durch Zugabe einer Mineralsäure wie Salzsäure, Bromwasserstoffsäure oder Schwefelsäure

oder einer organischen Säure wie Essigsäure oder Ameisensäure bei -10° bis +120°C vorzugsweise bei 0°C bis 30°C einen sauren pH-Wert einstellt.

Schließlich werden die Nitrile der Formel IV in an sich bekannter Weise unter sauren oder basischen Bedingungen, vorzugsweise mit Mineralsäuren wie Salzsäure, Bromwasserstoffsäure oder Schwefelsäure bei 0° bis 150°C, vorzugsweise bei 60° bis 120°C zu den erfindungsgemäßen Verbindungen der Formel I hydrolysiert.

Aus den entstandenen Aminosäuren können nach den üblichen Methoden der Peptidchemie die Ester hergestellt werden. Als günstig erwies es sich auch, die Nitrile der Formel IV unter Säurekatalyse (z. B. HCl) mit entsprechenden Alkoholen über die Iminoester zu den Estern umzusetzen (vgl. z. B. Org. Synth., Coll. Vol. 2 310 [1943]).

Die als Ausgangsmaterialien verwendeten Pyrrolidinderivate der Formel II sind zum großen Teil aus der Literatur bekannt oder werden nach im Prinzip bekannten Verfahren in wenigen Schritten hergestellt. So findet man beispielsweise Angaben über cis-Octahydroindol in der DE-A-23 02 198, über trans-Octahydroindol in Yakugaku Zasshi 95, 889 (1975), über cis-Octahydrocyclopenta[b]pyrrol in J. Org. Chem. 43, 54 (1978), über trans-Octahydrocyclopenta[b]pyrrol im SU-PS-761 462, über 2-Aza-spiro[4.4]nonan und 2-Azaspiro[4.5]decan in J. Med. Chem. 15, 129 (1972), über Spiro[bicyclo[2.2.1]-heptan-2,3'-pyrrolidin] in der DE-A-23 21 057, über 1-Aza-spiro[4.4]nonan im US-Patent 3 814 324, über Octahydroisoindol in Collect. Czech. Chem. Commun. 40, 3904 (1975), über Octahydrocyclopenta[c]pyrrol in der DE-A-24 15 064, über 2.3-Dimethylpyrrolidin in J. Organomet. Chem. 181, 255 (1979), über 3-Phenylpyrrolidin in Japan Kokai 74-72.266 sowie über eine Reihe von weiteren Derivaten in Arzneim.-Forsch. 12, 2089 (1971).

Die Verbindungen der Formel I sind wertvolle Zwischenprodukte bei der Herstellung von Pharmazeutika, insbesondere von Inhibitoren der "Angiotensin Converting Enzym" (ACE). Verbindungen dieses Typs sind beispielsweise aus der EP-A-50 800 bekannt oder sind auch Gegenstand der deutschen Patentanmeldung P 31 51 690.4. Solche ACE-Inhibitoren sind beispielsweise substituierte Acylderivate der Formel V,

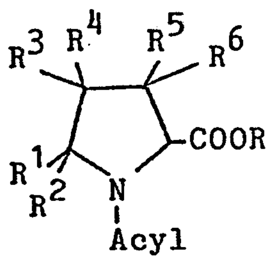

V

in welcher R, $R_1$ bis $R_4$ wie vorstehend definiert sind, $R^5$ und $R^6$ Wasserstoff bedeuten und Acyl z. B. für einen Rest der Formel VI steht,

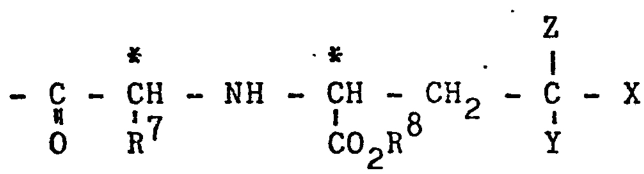

VI

worin

$R^7$= Wasserstoff, $(C_1-C_6)$-Alkyl, das gegebenenfalls durch Amino, $(C_1-C_4)$-Acylamino oder Benzoylamino substituiert sein kann, $(C_2-C_6)$-Alkenyl, $(C_5-C_9)$-Cycloalkyl, $(C_5-C_9)$-Cycloalkenyl, $(C_5-C_7)$-Cycloalkyl-$(C_1-C_4)$-alkyl, Aryl oder teilhydriertes Aryl, das jeweils durch $(C_1-C_2)$-Alkyl, $(C_1-C_2)$-Alkoxy oder Halogen substituiert sein kann, Aryl-$(C_1-C_4)$-alkyl, dessen Arylrest, wie vorstehend definiert substituiert sein kann, einen mono- bzw. bicyclischen Heterocyclenrest mit 5 bis 7 bzw. 8 bis 10 Ringatomen, wovon 1 bis 2 Ringatome Schwefel- oder Sauerstoffatome und/oder wovon 1 bis 4 Ringatome Stickstoffatome darstellen, oder eine Seitenkette einer Aminosäure,

$R^8$ = Wasserstoff, $(C_1-C_6)$-Alkyl, $(C_2-C_6)$-Alkenyl oder Aryl-$(C_1-C_4)$-alkyl,

Y= Wasserstoff

Z= Wasserstoff oder

Y und Z= zusammen Sauerstoff

X= $(C_1-C_6)$-Alkyl, $(C_2-C_6)$-Alkenyl, $(C_5-C_9)$-Cycloalkyl, Aryl, das durch $(C_1-C_4)$-Alkyl, $(C_1-C_4)$-Alkoxy, Hydroxy, Halogen, Nitro, Amino, $(C_1-C_4)$-Alkylamino, Di$(C_1-C_4)$alkylamino und/oder Methylendioxy mono-, di- oder trisubstituiert sein kann, oder Indol-3-yl bedeutet sowie deren physiologisch unbedenkliche Salze.

Verbindungen der Formel V können beispielsweise durch N-Acylierung von geeigneten Estern der Verbindungen der Formel I, wie beispielsweise Benzyl- oder tert.-Butylestern mit Verbindungen der Formel Acyl-OH, in der Acyl wie vorstehend definiert ist und anschließend hydrogenolytische, saure oder basische Abspaltung der Estergruppen hergestellt werden.

Die Kondensation von Estern der Verbindungen der Formel I mit Verbindungen der Formel Acyl-OH erfolgt vorzugsweise nach bekannten Methoden der Peptidchemie. Besonders bevorzugt sind solche Verfahren, die ausreichend Schutz von Racemisierung bieten, wie z. B. die DCC/HOBt-Methode oder die im US-Patent 43 31 592 beschriebene Alkanphosphonsäureanhydridmethode.

Die Verbindungen der Formel V besitzen eine lang andauernde, intensive blutdrucksenkende Wirkung. Sie werden nach peroraler Gabe gut resorbiert und können zur Bekämpfung des Bluthochdrucks verschiedener Genese eingesetzt und für sich oder in Kombination mit anderen blutdrucksenkenden, gefäßerweiternden oder diuretisch wirksamen Verbindungen angewandt werden. Die Anwendung kann intravenös, subcutan oder peroral erfolgen, die perorale Gabe wird bevorzugt. Die Dosierung liegt bei peroraler Gabe in der Regel bei 0.01 bis 10 mg/kg Tag.

Sie kann in schweren Fällen auch erhöht werden, da toxische Eigenschaften bisher nicht beobachtet wurden. Auch eine Herabsetzung der Dosis ist möglich und vor allem dann angebracht, wenn gleichzeitig Diuretika verabreicht werden. Bei intravenöser und subkutaner Gabe sollte die Einzeldosis zwischen 0.1 und 250 $\mu$g/Tag liegen.

Die folgenden Beispiele sollen die Erfindung erläutern, ohne sie auf die beschriebenen Beispiele zu beschänken.

Beispiel 1

Prolin

a) $\Delta^1$-Pyrrolin-Trimer

Eine Lösung von 4,05 g (0,015 Mol) Kaliumperoxodisulfat in 20 ml Wasser wird zu einer gerührten Mischung von 1,06 g (0,015 Mol) Pyrrolidin, 1,2 g (0,03 Mol) Natriumhydroxid und 12,7 mg (0,075 mmol) Silbernitrat in 15 ml Wasser bei 0°C getropft und 2,5 Stunden gerührt. Nach Sättigen mit Natriumchlorid wird mit Dichlormethan extrahiert, über Natriumsulfat getrocknet und bei 0° am Rotationsverdampfer eingeengt. Man erhält 0,53 g Titelverbindung als Öl.

$^1$H-NMR-Daten ($CDCl_3$) : $\delta$= 1,2 - 2,1 (m, 12 H)
2,1 - 2,5 (m, 3 H)
3,0 - 3,2 (m, 6 H) ppm.

b) 2-Cyano-pyrrolidin

0,53g $\Delta^1$-Pyrrolin-Trimer (0,0075 Mol) werden in 5 ml $H_2O$ suspendiert, 0,5 g Kaliumcyanid werden zugesetzt und bei 0°C anschließend 5 ml 2 N Salzsäure zugetropft. Nach 15 Stunden bei Raumtemperatur wird mit Essigester extrahiert, die wäßrige Phase mit 2 N NaOH alkalisch gestellt und mit $CH_2Cl_2$ extrahiert. Der Dichlormethan-extrakt wird über Natriumsulfat getrocknet und eingeengt. Man erhält 0.4 g Öl.

$^1$H-NMR-Daten ($CDCl_3$) : $\delta$= 3,8 - 3,6 (m, 1 H)
3,4 - 3,0 (m, 2 H)
1,8 - 1,2 (m, 4 H) ppm.

c) Prolin

0,4 g 2-Cyanopyrrolidin werden in 5 ml 5 N Salzsäure gelöst und 2 Stunden am Rückfluß gekocht. Nach Abkühlen wird eingeengt, in $H_2O$ aufgenommen, mit Amberlite® IRA 93 (OH-Form) auf pH 5 gestellt, filtriert und eingeengt. Verreiben mit Isopropylether ergibt 0,3 g Prolin. Schmp. 210°C (Zers.)

Beispiel 2

cis-Octahydroindol-2-carbonsäure

a) cis-3,3a,4,5,6,7,7a-Hexahydroindol

6 g (0,048 Mol) cis-Octahydroindol werden mit 13,5 g (0,05 Mol) Kaliumperoxodisulfat, 4 g Natriumhydroxid und 0,1 g Silbernitrat in 200 ml Wasser nach dem in Beispiel 1a beschriebenen Verfahren umgesetzt. Man erhält 4 g der Titelverbindung als Öl.

$^1$H-NMR-Daten ($CDCl_3$) : $\delta$= 7,3 (br. s. 1 H)
4,6 - 4,3 (m, 1 H)
4,0 - 1,3 (m, 11 H) ppm.

b) 2-Cyano-cis-octahydroindol

Nach dem in Beispiel 1 b beschriebenen Verfahren werden 4g cis-3,3a,4,5,6,7,7a-Hexahydroindol mit 2,1 g Kaliumcyanid und 21 ml 2 N Salzsäure umgesetzt. Man erhält 3.9 g der Titelverbindung als Öl.

$^1$H-NMR-Daten ($CDCl_3$) : $\delta$= 4,2 - 3,5 (m, 2 H)
3,0 - 1,1 (m, 11 H) ppm.

c) cis-Octahydroindol-2-carbonsäure

3,9 g 2-Cyano-cis-octahydroindol werden mit 5 N Salzsäure (50 ml) nach dem in Beispiel 1c beschriebene Verfahren umgesetzt. Man erhält ein 2:1-Gemisch aus cis-Octahydroindol-2-exo-carbonsäure (2A) und cis-Octahydroindol-2-endo-carbonsäure (2B), das durch Chromatographie an Kieselgel und Kristallisation aus Ethanol/Aceton getrennt werden kann. Rf-Werte ($SiO_2$, Laufmittel $CH_2Cl_2$/MeOH/HOAc/$H_2O$= 10:5:1:1)

2A: 0.67
2B: 0.62

Schmelzpunkte : 2A : amorphes Pulver.
2B : 230 - 233°C

Beispiel 3

trans-Octahydroindol-2-carbonsäure

Nach den in Beispiel 1a - c beschriebenen Verfahrensschritten erhält man aus trans-Octahydroindol (4,5 g) 2 g eines 1:1-Gemisches aus trans-Octahydroindol-2-α-carbonsäure (3A) und trans-Octahydroindol-2-ß-carbonsäure (3B), Schmp. 280°C. Die Isomeren können durch Kristallisation aus Ethanol/Aceton getrennt werden.

Beispiel 4

cis-Octahydrocyclopenta[b]pyrrol-2-carbonsäure

Nach dem in Beispiel 1a - c beschriebenen Verfahrensschritten erhält man aus cis-Octahydrocyclopenta[b]pyrrol ein 2,7:1-Gemisch aus cis-Octahydrocyclopenta[b]pyrrol-2-exo-carbonsäure (4A) und cis-Octahydrocyclopenta[b]pyrrol-2-endocarbonsäure (4B). Nach Aufnehmen mit Aceton kristallisiert das reine 4A aus, Schmp. >180°C (Zers.)

Aus der Mutterlauge der Kristallisation kann reines 4B vom Schmp. 205 - 209°C Hydrochlorid gewonnen werden.

Beispiel 5

trans-Octahydrocyclopenta/b/pyrrol-2-carbonsäure

Nach dem in Beispiel 1a - c beschriebenen Verfahren wird trans-Octahydrocyclopenta/b/pyrrol zu einem 1:1-Gemisch aus trans-Octahydrocyclopenta/b/pyrrol-2-α-carbonsäure (5A) und trans-Octahydrocyclopenta/b/pyrrol-2-ß-carbonsäure (5B) umsetzt. Durch Kristallisation aus Aceton/Ethanol kann 5B rein erhalten werden, Schmp. >250°C (Zers.)

Ausbeute 35 %

Beispiel 6

2-Azaspiro/4,5/decan-3-carbonsäure

Nach dem in Beispiel 1a - c beschriebene Verfahren wird aus 2-Azaspiro/4,5/decan die Titelverbindung im Gemisch mit geringeren Mengen 2-Azaspiro/4,5/decan-1-carbonsäure erhalten, aus dem sie durch Kristallisation aus Ethanol/Aceton rein abgetrennt werden kann.

Ausbeute 30 %
Schmp. 205°C (Zers.)

Beispiel 7

2-Azaspiro/4,4/nonan-3-carbonsäure

Nach dem in Beispiel 1 a - c beschriebenen Verfahren wird die Titelverbindung aus 2-Aza-spiro/4,4/nonan im Gemisch mit geringen Mengen 2-Azaspiro/4,4/nonan-1-carbonsäure erhalten, durch Kristallisation mit Ethanol/Aceton kann sie rein erhalten werden.

Ausbeute 27 %
amorphes Pulver

$^1$H-NMR-Daten ($D_2O$) : 4,02 (t, 1 H)
2,3 (s, 2 H)
2,0 - 1,1 (m, 1 OH) ppm

Beispiel 8

5'-Cyano-spiro/bicyclo/2.2.2/octan-2,3'-pyrrolidin/

a) Spiro/bicyclo/2.2.2/octan-2,3'-pyrrolin-Δ3'/

Nach den in Beispiel 1a beschriebenen Verfahren erhält man aus 4,3 g Spiro/bicyclo/2.2.2/octan-2,3'-pyrrolidin/ 3,0 g der Titelverbindung als Öl im Gemisch mit ihrem Trimeren.

b) 5'-Cyano-spiro/bicyclo/2.2.2/octan-2,3'-pyrrolidin/

3 g der Verbindung aus 8a werden zusammen mit 2.25 g Kaliumcyanid in 100 ml Wasser gelöst. Unter Eiskühlung tropft man 24 ml 2 N Salzsäure zu und rührt 72 Stunden bei Raumtemperatur. Nach Extraktion mit Essigester wird die wäßrige Lösung mit 1 N NaOH alkalisch gestellt, mit Ether extrahiert und die Extrakte über Natriumsulfat getrocknet. Nach Einengen wird das Rohprodukt an Kieselgel mit Essigester/Cyclohexan (1:1) als Laufmittel chromatographiert, wodurch die endo und exo-Isomeren getrennt werden.

endo-Isomer (8bA) : 0,83 g, Schmp. 78 - 80°C

$^1$H-NMR-Daten ($CDCl_3$) : $\delta$= 4,0 (t. J= 14 Hz, 1 H)
2,8 (s, 2 H)
2,4 (s, 1 H)
2,0 (d, 2 H)
1,5 (br. s, 12 H) ppm.

exo-Isomer (8bB) : 1,5 g, Schmp. 38 - 40°C

$^1$H-NMR-Daten ($CDCl_3$) : $\delta$= 4,02 (X-Teil eines ABX-Systems, 1 H
2,85 (AB-System, J= 15 Hz, 2 H)
2,5 - 1,0 (m, 15 H) ppm.

Beispiel 9

Spiro/bicyclo/2.2.2/octan-2,3'-pyrrolidin/-5'-endo-carbonsäure

Nach dem in Beispiel 1c beschriebenen Verfahren erhält man aus 0,8 g der Verbindung 8bA 0.9 g der Titelverbindung, Schmp. 236°C.

$^1$H-NMR-Daten ($D_2O$) : $\delta$= 4,2 (X-Teil eines ABX-Systems, 1 H)
3,2 (s, 2 H)
2,5 - 1,5 (AB-Teil eines ABX-Systems, 2 H)
1,5 (br. s, 12 H) ppm

Massenspektrum (m/e) : 209 ($M^+$, 0,8 %), 165 (13 %), 164 (M-COOH, 100 %), 87 (14 %), 69 (10 %).

Beispiel 10

Spiro/bicyclo/2.2.2/octan-2,3'-pyrrolidin/-5'-exo-carbonsäure

Aus 1,5 g der Verbindung 8bB erhält man 1,4 g der Titelverbindung nach dem in Beispiel 1c beschriebenen Verfahren, Schmp. 242°C.

$^1$H-NMR-Daten ($D_2O$) : $\delta$= 4,15 (X-Teil eines ABX-Systems, 1 H).
3,2 (AB-System, J= 15 Hz, 1 H)
2,5 - 1,7 (AB-Teil eines ABX-Systems, 2 H)
1,5 (br. s, 12 H) ppm

Beispiel 11

Spiro/bicyclo/2.2.1/heptan-2,3'-pyrrolidin/-5'-exo-carbonsäure

Nach dem in Beispiel 1a - c beschriebenen Verfahren erhält man aus Spiro/bicyclo/2.2.1/heptan-2,3'-pyrrolidin/ die Titelverbindung als Isomerengemisch, farbloses amorphes Pulver.

Ausbeute 24 %

$^1H$-NMR-Daten ($D_2O$) : $\delta$ = 4,3 - 4,0 (m, 1 H)
3,5 (m, AB-System, 2 H)
2,5 - 1,0 (m, 12 H) ppm.

Beispiel 12

cis-exo-3-Azatricyclo[5.2.1.0$^{2.6}$]decan-4-carbonsäure

Nach den in den Beispielen 1a und b beschriebenen Verfahren erhält man aus cis-exo-3-Azatricyclo[5.2.1.0$^{2.6}$]decan ein 1:1-Gemisch aus 4-exo-Cyano-cis-exo-3-azatricyclo-[5.2.1.0$^{2.6}$]decan und 4-endo-Cyano-cis-exo-3-azatricyclo[5.2.1.0$^{2.6}$]decan, die durch Chromatographie an Kieselgel getrennt und nach dem in Beispiel 1c beschriebenen Verfahren zu den exo- bzw. endo-Isomeren der Titelverbindung hydrolysiert werden

exo-Isomer: $R_f$ 0,54 (Kieselgel; $CH_2Cl_2$/MeOH/HOAc/-$H_2O$= 20:15:2:4)

endo-Isomer: $R_f$ 0.51 (Kieselgel; $CH_2Cl_2$/MeOH/HOAc/-$H_2O$= 20:15:2:4)

Beispiel 13

cis-endo-3-Azatricyclo[5.2.1.0$^{2.6}$]decan-4-carbonsäure

Wie im Beispiel 12 beschrieben und unter Verwendung der in Beispiel 1a - c beschriebenen Verfahren erhält man aus cis-endo-3-Azatricyclo[5.2.1.0$^{2.6}$]decan die exo- und endo-Isomeren der Titelverbindung im Verhältnis von 8:1.

exo-Isomer: $R_f$ 0,61 (Kieselgel; $CH_2Cl_2$/MeOH/HOAc/-$H_2O$= 20:15:2:4)

endo-Isomer: $R_f$ 0.66 (Kieselgel; $CH_2Cl_2$/MeOH/HOAc/-$H_2O$= 20:15:2:4)

Beispiel 14

cis-Decahydrocyclohepta/b7pyrrol-2-carbonsäure

Nach den in den Beispielen 1a bis c beschriebenen Verfahren erhält man aus 5,2 g cis-Decahydro-2-azaazulen ein 2:1-Gemisch der exo- und endo-Isomeren der Titelverbindung, die durch Kristallisation aus Ethanol/Aceton oder Chromatographie der Nitrilvorstufe an Kieselgel getrennt werden können.

exo-Isomer (14A) amorphes Pulver

endo-Isomer (14B) farblose Kristalle, Schmp. 252 - 256°C

Beispiel 15

trans-Decahydrocyclohepta/b7pyrrol-2-carbonsäure

Nach dem in Beispiel 1a bis c beschriebene Verfahren erhält man aus trans-Decahydro-2-aza-azulen die Titelverbindung als nicht trennbares 1:1-Gemisch der α- und ß-Isomeren.

Beispiel 16

cis-Octahydroisoindol-1-carbonsäure

Nach den in Beispiel 1a und b beschriebenen Verfahren erhält man aus 0,8 g cis-Octahydroisoindol ein Gemisch aus 5 Teilen 1-exo-Cyano-cis-octahydroisoindol (NMR ($CDCl_3$) : $\delta$= 3,8 (d, 1 H)) und 1 Teil 1-endo-Cyano-cis-octahydroisoindol (NMR ($CDCl_3$) : $\delta$= 4,0 (d, 1 H).

Nach Trennung der Isomeren an Kieselgel mit Essigester/Cyclohexan (1:1) als Laufmittel werden diese nach den in Beispiel 1c beschriebenen Verfahren verseift. Man erhält 0,3 g cis-Octahydroisoindol-1-exo-carbonsäure (16A) und 0,05 g cis-Octahydroisoindol-1-endo-carbonsäure (16B) jeweils als farblose Pulver.

Beispiel 17

trans-Octahydroisoindol-1-carbonsäure

Nach den in den Beispiel 1a - c beschriebenen Verfahren erhält man ein 1:1-Gemisch aus trans-Octahydroisoindol-1-α-carbonsäure (17A) und trans-Octahydroisoindol-1-ß-carbonsäure (17B) ausgehend von trans-Octahydroisoindol in 27 %iger Ausbeute.
$R_f$= 0,65 ($SiO_2$; $CH_2Cl_2$/MeOH/HOAc/$H_2O$= 10:5:1:1)

Beispiel 18

cis-Octahydrocyclopenta[c]pyrrol-1-exo-carbonsäure

Nach den in Beispiel 1a - c beschriebenen Verfahren erhält man ausgehend von cis-Octahydrocyclopenta[c]pyrrol die Titelverbindung in 34 % Ausbeute.

Schmp. 190 - 195°C nach Kristallisation aus Ethanol/Diisopropylether.

Beispiel 19

1-Aza-spiro[4.5]decan-2-carbonsäure

Nach dem in Beispiel 1a - c beschriebenen Verfahren erhält man aus 1-Aza-spiro[4.5]decan die Titelverbindung in 30 % Ausbeute. Farblose Kristalle vom Schmp. 129 - 132°C (aus Aceton/Diisopropylether)

Beispiel 20

1-Aza-spiro[4.4]nonan-2-carbonsäure

Nach dem in Beispiel 1a - c beschriebenen Verfahren erhält man aus 1-Azaspiro[4.4]nonan die Titelverbindung in 18 % Ausbeute als farbloses amorphes Pulver.

Beispiel 21

4,5-cis-Diethylprolin

Nach den in Beispiel 1 a - c beschriebenen Verfahren erhält man aus 4,5-cis-Diethylpyrrolidin nach chromatographischer Trennung der Nitrilstufen und getrennter salzsaurer Hydrolyse die Isomeren mit der Carboxygruppe in cis-Stellung zu den Ethylgruppen (21A), Schmp. 230 - 35°C (Zers.)) sowie in trans-Stellung zu den Ethylgruppen (21B, Schmp. 158 - 162°C).

Patentansprüche:

1. Verfahren zur Herstellung von einer Verbindung der Formel I

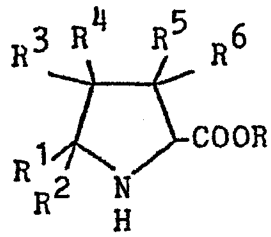

(I)

in welcher

R für Wasserstoff, $(C_1-C_6)$-Alkyl oder $(C_7-C_9)$-Aralkyl steht, und

$R^1$ bis $R^6$ gleich oder verschieden sind und unabhängig voneinander Wasserstoff, $(C_1-C_8)$-Alkyl, $(C_3-C_9)$-Cycloalkyl, $(C_3-C_9)$-Cycloalkyl-$(C_1-C_4)$-alkyl, $(C_5-C_9)$-Cycloalkenyl-$(C_1-C_4)$-alkyl, $(C_6-C_{12})$-Aryl-$(C_1-C_4)$-alkyl oder $(C_6-C_{12})$-Aryl, die beide jeweils im Arylteil durch $(C_1-C_4)$-Alkyl, $(C_1-C_4)$-Alkoxy, Hydroxy, Halogen, Nitro, Methylendioxy und/oder Cyano mono-, di- oder trisubstituiert sein können, bedeuten

oder in welcher

zwei der Reste $R^1$ bis $R^6$ zusammen mit dem sie tragenden Kohlenstoffatom bzw. mit den beiden sie tragenden Kohlenstoffatomen ein 4- bis 10-gliedriges gesättigtes oder ungesättigtes, mono- oder bicyclisches carbocyclisches Ringsystem bilden und die übrigen Reste Wasserstoff sind, dadurch gekennzeichnet, daß man ein Pyrrolidinderivat der Formel II,

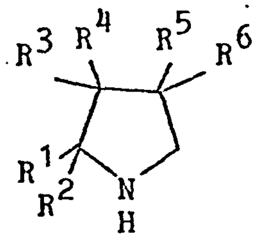

(II)

in welcher $R^1$ bis $R^6$ die gleiche Bedeutung wie in Formel I haben, mit einem Oxidationsmittel in Gegenwart eines Silbersalzes in ein $\Delta^1$-Pyrrolinderivat der Formel III,

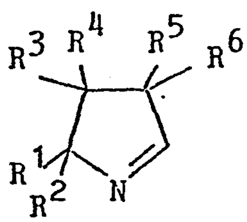

(III)

in welcher $R^1$ bis $R^6$ die gleiche Bedeutung wie in Formel I haben, überführt, dieses mit Cyanwasserstoff oder einem Metallcyanid zu einer Verbindung der Formel IV,

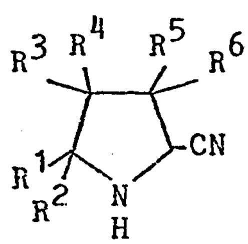

(IV)

in welcher $R^1$ bis $R^6$ die gleiche Bedeutung wie in Formel I haben, umsetzt, und diese mit einer Verbindung der Formel ROH, in welcher R die oben definierte Bedeutung hat, unter Bildung einer Verbindung der Formel I umsetzt.

2. Verfahren gemäß Anspruch 1, dadurch gekennzeichnet, daß eine Verbindung der Formel I hergestellt wird, in welcher

R die in Anspruch 1 definierte Bedeutung hat und $R^1$ bis $R^6$ gleich oder verschieden sind und unabhängig voneinander Wasserstoff, Methyl, Ethyl, Propyl, Isopropyl, tert. Butyl, Cyclopentyl, Cyclohexyl, Cycloheptyl, Cyclopentylmethyl, Cyclopentylethyl, Cyclohexylmethyl, Phenyl, Naphthyl, 4-Methoxyphenyl, 4-Fluorphenyl, 4-Chlorphenyl, 3,4-Dimethoxyphenyl, 3,4-Methylendioxyphenyl, 3,4-Dichlorphenyl, p-Tolyl, Benzyl, 4-Methoxybenzyl, 4-Chlorbenzyl, Phenylethyl, 2-Phenylpropyl oder 1-Phenylpropyl, bedeuten

oder in welcher zwei der Reste $R^1$ bis $R^6$ in der im Anspruch 1 definierten Weise einen Cyclobutyl-, Cyclopentyl-, Cyclohexyl-, Cycloheptyl-, Cyclooctyl-, Bicyclo[2.2.1]heptyl- oder Bicyclo[2.2.2]octyl-Ring bilden, wobei die Bindungen vom gleichen Kohlenstoffatom oder von benachbarten Kohlenstoffatomen ausgehen und wobei die übrigen Reste Wasserstoff sind.

3. Verfahren gemäß Anspruch 1, dadurch gekennzeichnet, daß eine Verbindung der Formel I hergestellt wird, in welcher R die im Anspruch 1 definierte Bedeutung hat und $R^1$ bis $R^6$ Wasserstoff bedeuten oder worin einer oder zwei der Reste $R^1$ bis $R^6$ unabhängig voneinander Methyl, Ethyl, Propyl, Isopropyl, Cyclopentyl, Cyclohexyl, Phenyl, 4-Methoxyphenyl, 4-Fluorphenyl, Benzyl, Phenethyl oder 4-Methoxybenzyl und die übrigen Wasserstoff bedeuten oder zwei der Reste $R^1$ bis $R^6$, die am gleichen oder an benachbarten Kohlenstoffatomen stehen, mit diesen zusammen einen Cyclopentyl-, Cyclohexyl-, Cycloheptyl-, Bicyclo[2.2.1]heptyl oder Bicyclo[2.2.2]octyl-Ring bilden, wobei die übrigen Reste Wasserstoff bedeuten.

4. Verfahren gemäß einem der Ansprüche 1 bis 3, dadurch gekennzeichnet, daß die Oxidation der Verbindung der Formel II zur Verbindung der Formel III mit einem Peroxodisulfat durchgeführt wird.

5. Verfahren gemäß einem der Ansprüche 1 bis 4, dadurch gekennzeichnet, daß man eine Verbindung der Formel IV mit Wasser umsetzt.

6. Verfahren gemäß einem der Ansprüche 1 bis 4, dadurch gekennzeichnet, daß man eine Verbindung der Formel IV mit einer Verbindung der Formel ROH, in der R die in Anspruch 1 definierten Bedeutungen mit Ausnahme der von Wasserstoff hat, in Gegenwart einer Säure umsetzt.

Europäisches Patentamt

# EUROPÄISCHER RECHERCHENBERICHT

0132580

Nummer der Anmeldung

EP 84 10 6954

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl ³) |
|---|---|---|---|
| D,X | EP-A-0 050 800 (SCHERING CORP.) * Seiten 1-15 * ----- | 1-6 | C 07 D 207/16<br>C 07 D 209/42<br>C 07 D 209/52<br>C 07 D 209/54 |
| | | | **RECHERCHIERTE SACHGEBIETE (Int. Cl. ³)** |
| | | | C 07 D 207/00<br>C 07 D 209/00 |

Der vorliegende Recherchenbericht wurde für alle Patentansprüche erstellt.

| Recherchenort | Abschlußdatum der Recherche | Prüfer |
|---|---|---|
| DEN HAAG | 23-10-1984 | MAISONNEUVE J.A. |

KATEGORIE DER GENANNTEN DOKUMENTEN

- X : von besonderer Bedeutung allein betrachtet
- Y : von besonderer Bedeutung in Verbindung mit einer anderen Veröffentlichung derselben Kategorie
- A : technologischer Hintergrund
- O : nichtschriftliche Offenbarung
- P : Zwischenliteratur
- T : der Erfindung zugrunde liegende Theorien oder Grundsätze
- E : älteres Patentdokument, das jedoch erst am oder nach dem Anmeldedatum veröffentlicht worden ist
- D : in der Anmeldung angeführtes Dokument
- L : aus andern Gründen angeführtes Dokument
- & : Mitglied der gleichen Patentfamilie, übereinstimmendes Dokument